# FASCICULE DE BREVET EUROPEEN

(11) **EP 0 573 362 B1**
(45) Date de publication et mention de la délivrance du brevet: **23.10.1996**
(21) Numéro de dépôt: 93401419.2
(22) Date de dépôt: 03.06.1993
(51) Int. Cl.: C07J 5/00, C07J 7/00

(54) **Nouveau procédé de préparation d'un dérivé stéroide 11-céto**
Neues Verfahren zur Herstellung 11-Keto Steroidderivate
New process for the production of 11-keto steroids

(30) Priorité: 04.06.1992 FR 9206773
(43) Date de publication de la demande: 08.12.1993
(73) Titulaire: ROUSSEL UCLAF, 93230 Romainville (FR)
(72) Inventeur: Brion, Francis, F-93220 Gagny (FR); Buendia, Jean, F-94170 Le Perreux Sur Marne (FR); Diolez, Christian, F-91000 Palaiseau (FR); Vivat, Michel, F-77400 Lagny Sur Marne (FR)
(74) Mandataire: Vieillefosse, Jean-Claude

(56) Documents cités:
- EP-A- 0 030 368
- EP-A- 0 097 328
- EP-A- 0 531 212
- TETRAHEDRON LETTERS, no. 37, juillet 1968, Oxford (GB); S.L. NEIDLEMAN et al., pp. 4057-4059

## Description

La présente invention concerne un nouveau procédé de préparation d'un dérivé stéroïde 11-céto.

L'invention a ainsi pour objet un procédé de préparation des composés de formule (I) 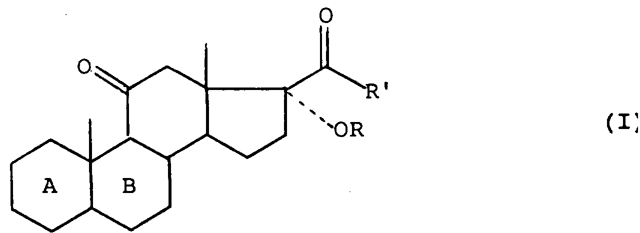 dans laquelle R représente un atome d'hydrogène ou un reste d'ester ou d'éther, R' représente un radical méthyle ou un radical -CH₂OR'', R'' représentant un reste d'ester d'hydrogène ou d'éther identique ou différent de R, et les cycles A et B représentent un reste : 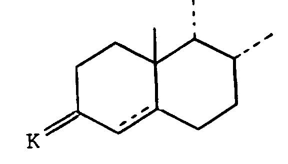 dans lequel K représente un atome d'oxygène ou un groupement protecteur du radical 3-oxo, de formule 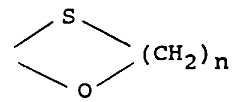 ou 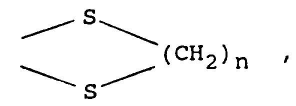 dans laquelle n est égal à 2 ou 3 et le trait pointillé représente une éventuelle seconde liaison, qui consiste à transformer un composé de formule (II) : 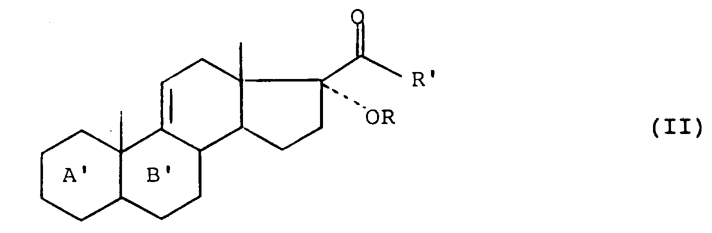 dans laquelle R et R' sont définis comme précédemment et les cycles A' et B' représentent soit les cycles A et B tels que définis précédemment, soit l'un des restes suivants : 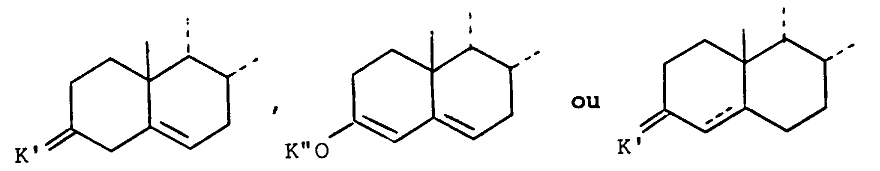 K' représentant un groupement protecteur de formule 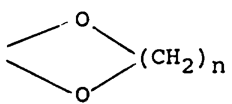 dans laquelle n est défini comme précédemment et K'' représente un radical alkyle renfermant de 1 à 8 atomes de carbone ou un radical aryle renfermant de 6 à 12 atomes de carbone, en une halohydrine de formule (III) : 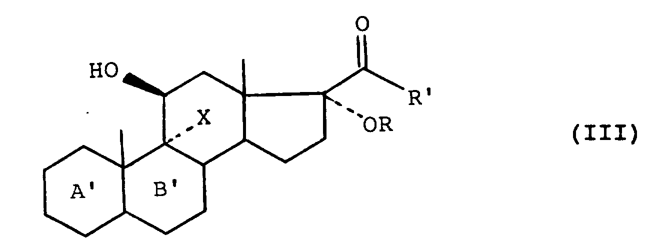 dans laquelle X représente un atome d'halogène et R, R', A' et B' sont définis comme précédemment, et est caractérisé en ce que l'on soumet ladite halohydrine à une réaction de réarrangement en présence d'un alcool, pour obtenir après traitement par un acide le composé de formule (I) attendu.

Par atome d'halogène, on entend de préférence un atome de chlore, de brome ou d'iode, le brome étant plus particulièrement préféré.

Lorsque R et R'' représentent un reste d'ester, il s'agit de préférence d'un radical acyle renfermant de 1 à 8 atomes de carbone et plus particulièrement d'un radical formyle, acétyle, propionyle, butyryle, valéryle ou benzoyle.

Lorsque R et R'' représentent un reste d'éther, il s'agit de préférence d'un radical alkyle renfermant de 1 à 6 atomes de carbone, par exemple méthyle, éthyle ou propyle, d'un radical tétrahydropyranyle ou d'un reste d'éther silylé, par exemple trialkylsilyle tel que triméthyl- ou diméthylterbutylsilyle, triarylsilyle tel que triphénylsilyle ou diarylalkylsilyle tel que diphénylterbutylsilyle.

Lorsque K" représente un radical alkyle, il s'agit de préférence d'un radical méthyle ou éthyle.

Lorsque K" représente un radical aryle, il s'agit de préférence d'un radical phényle.

L'invention a notamment pour objet un procédé caractérisé en ce que la réaction de réarrangement est effectuée en présence d'un alcool supérieur ou d'un polyalcool.

L'alcool supérieur ou le polyalcool est de préférence choisi dans le groupe constitué par le glycérol, l'éthylène glycol et le propylène glycol, l'éthylène glycol étant tout particulièrement préféré.

On opère de préférence en présence de l'alcool ou du polyalcool en excès, par chauffage à une température inférieure à 100°C. Par excès, on entend préférentiellement de 10 à 20 équivalents.

En outre, on opère avantageusement en présence d'un cosolvant, inerte dans les conditions de la réaction, de point d'ébullition inférieur à 100°C, et au reflux de celui-ci.

Le cosolvant est par exemple un ester tel que l'acétate d'éthyle, le benzène ou le cyclohexane. L'acétate d'éthyle est plus particulièrement préféré.

Le traitement acide subséquent est de préférence réalisé par un acide minéral ou organique aqueux, par exemple par l'acide chlorhydrique, bromhydrique, sulfurique, perchlorique, nitrique, paratoluènesulfonique, acétique, formique, oxalique, ou encore par une résine acide.

Le traitement acide a notamment pour conséquence de débloquer le cétal formé intermédiairement en 20 et, le cas échéant, en 3, par l'action de l'alcool. Il a aussi pour conséquence, lorsque l'on utilise au départ du procédé un composé de formule (II) dans laquelle les cycles A' et B' sont différents de A et B et seulement dans ce cas, de libérer le système 3-céto Δ4 ou la fonction 3-céto.

L'invention a notamment pour objet un procédé tel que défini précédemment, caractérisé en ce que l'on met en oeuvre une halohydrine de formule (III) dans laquelle X représente un atome de brome.

L'invention a aussi notamment pour objet un procédé tel que défini précédemment, caractérisé en ce que l'on met en oeuvre une halohydrine de formule (III) dans laquelle les cycles A' et B' représentent un reste de formule : 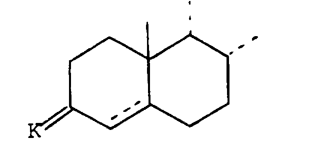 dans laquelle K et le trait pointillé en 4(5) sont définis comme précédemment.

L'invention a encore notamment pour objet un procédé tel que défini précédemment, caractérisé en ce que l'on met en oeuvre une halohydrine de formule (III) dans laquelle R est choisi dans le groupe constitué par un atome d'hydrogène, un radical acyle renfermant de 1 à 8 atomes de carbone, un radical alkyle renfermant de 1 à 6 atomes de carbone, un radical tétrahydropyranyle et un reste d'éther silylé et R représente un radical méthyle ou un radical -CH₂-OR'', R'' identique ou différent de R, ayant l'une des valeurs de R indiquées ci-dessus.

Un procédé de transformation d'un stéroïde 9α-halo 11β-hydroxy en dérivé 11-céto a déjà été décrit dans la littérature (voir notamment le brevet européen 30368). Ce procédé consiste à chauffer l'halohydrine à une température élevée (180 à 350°C et, en pratique 250 à 300°C) en présence d'un solvant aprotique à haut point d'ébullition, pendant un court instant (quelques minutes).

Le solvant est notamment de type biphényle, dibenzofuranne, oxyde de diphénylène ou encore des éthers méthyliques de polyalcools.

Le procédé de la présente invention requiert des conditions opératoires beaucoup plus douces, possibles seulement en raison de la mise en oeuvre d'intermédiaires bloqués in situ au niveau des fonctions cétones. A titre purement indicatif, on peut préciser que la conséquence du blocage paraît être la labilisation de la liaison carbone halogène en 9, ce qui facilite le réarrangement et permet donc l'emploi de conditions opératoires très douces. En pratique, ceci correspond à opérer à une température inférieure à 100°C, et est réalisé grâce à l'utilisation d'un cosolvant approprié.

Le procédé du brevet européen 30368 ne prévoit pas la formation des intermédiaires mis en oeuvre dans le procédé de l'invention, c'est pourquoi il requiert des conditions opératoires différentes, faute de quoi, il ne conduirait pas au résultat recherché. En outre, il est évident qu'un tel procédé qui oblige à chauffer très fortement pendant un court instant l'halohydrine n'est guère envisageable au niveau industriel où des tonnages importants sont mis en jeu. Au contraire, le procédé de la présente invention permet aisément la synthèse industrielle. En outre, mettant en oeuvre des conditions beaucoup plus douces que celles décrites dans le brevet européen 30368, ceci représente un avantage au niveau du rendement de la réaction, car la formation de produits secondaires ou de dégradation est forcément limitée, donc aussi un avantage au niveau industriel, dans la mesure où la synthèse est d'autant plus économique.

Les halohydrines de formule (III) sont connues de manière générale ou peuvent être aisément préparés par des procédés connus de l'homme du métier. A titre d'exemples, on peut citer les références suivantes : J.A.C.S. 79 1135 (1957),
USP 2 763 671, 2 852 511 ou 2 963 498, BF 1 188 434,
USP 3 100 210, 3 084 174 ou 3 499 081, EP 97 328 ou 3341 ou encore DD 268 955. Les composés de formule (II) sont de même connus ou préparables selon des procédés connus.

Les composés de formule (I) sont soit des composés thérapeutiquement actifs connus, soit des intermédiaires connus pour préparer des composés thérapeutiquement actifs.

Les exemples suivants illustrent l'invention sans toutefois la limiter.

### EXEMPLE 1 : 17α-hydroxy pregn-4-èn-3,11,20-trione.

### STADE A : 9α-bromo 11β-17α-dihydroxy pregn-4-èn-3,20-dione.

On mélange sous gaz inerte 2 g de 17α-hydroxy pregna 4,9(11)-dièn-3,20-dione et 10 cm³ de tétrahydrofuranne, puis ajoute à 0°C 1,3 g de N-bromosuccinimide. On refroidit vers -3°C puis ajoute lentement un mélange de 1,3 cm³ d'acide perchlorique à 65% et 2,5 cm³ d'eau. On maintient sous agitation pendant 3 heures 30 minutes puis verse dans 100 cm³ de mélange eau-glace. On filtre les cristaux, les lave à l'eau et les sèche. On obtient 2,5 g de produit attendu, utilisé tel quel.

### STADE B : 17α-hydroxy pregn-4-èn-3,11,20-trione.

On mélange sous gaz inerte 2,05 g de 9α-bromo 11β-17α-dihydroxy pregn-4-èn-3,20-dione, 14 cm³ d'acétate d'éthyle et 4,6 cm³ d'éthylène glycol. On porte au reflux pendant 16 heures puis refroidit à 20°C. On ajoute alors 3,2 cm³ d'acide chlorhydrique concentré et 35 cm³ d'eau puis agite pendant 20 heures. On distille ensuite l'acétate d'éthyle sous pression réduite et ajoute 7 g de chlorure de sodium. On agite pendant 30 minutes à 20°C puis essore les cristaux et les lave à l'eau salée. On les reprend ensuite dans du chlorure de méthylène, sèche la solution et concentre à sec. On chromatographie le résidu sur silice en éluant au mélange chlorure de méthylène-acétate d'éthyle (8-2) et obtient 1,26 g de produit attendu.

### Spectre IR (CHCl₃) :

Absorptions à 3610 cm⁻¹ (OH) ; 1706-1667 cm⁻¹ (cétone conjuguée) ; 1617 cm⁻¹ (C=C).

### EXEMPLE 2 : 17α-acétoxy pregn-4-èn-3,11,20-trione. STADE A : 9α-bromo 11β-hydroxy 17β-acétoxy pregn-4-èn-3,20-dione.

On mélange sous gaz inerte 3,7 g de 17α-acétoxy pregna 4,9(11)-dièn-3,20-dione et 18,5 cm³ de tétrahydrofuranne, puis refroidit à 0°/-5°C et ajoute 2,18 g de N-bromosuccinimide. On ajoute ensuite lentement un mélange de 1,7 cm³ d'acide perchlorique à 70% et 3,4 cm³ d'eau puis maintient sous agitation pendant 1 heure. On verse ensuite dans 100 volumes d'un mélange eau-glace, filtre les cristaux, les lave à l'eau et les sèche. On obtient 4,82 g de produit attendu.

### Spectre IR (CHCl₃) :

Absorptions à 3612 cm⁻¹ (OH) ; 1731 cm⁻¹ (OAc) ; 1716 et 1354 cm⁻¹ (-CO-CH₃) ; 1662 et 1621cm⁻¹ (Δ4 3-one).

### STADE B : 17α-acétoxy pregn-4-èn-3,11,20-trione.

On porte au reflux sous gaz inerte, un mélange de 1 g de produit obtenu au stade A, 7 cm³ d'acétate d'éthyle et 2,3 cm³ d'éthylène glycol. On maintient au reflux pendant 24 heures puis refroidit à 20°C et ajoute 1,6 cm³ d'acide chlorhydrique concentré et 17,5 cm³ d'eau. On agite pendant 16 heures puis distille l'acétate d'éthyle sous pression réduite. On sature au chlorure de sodium puis extrait au chlorure de méthylène. On lave la phase organique par une solution aqueuse saturée de bicarbonate de sodium puis à l'eau saturée de chlorure de sodium et la sèche. On concentre à sec et chromatographie le résidu sur silice en éluant au mélange chlorure de méthylène-acétate d'éthyle (9-1). On obtient 0,51 g de produit attendu que l'on peut recristalliser dans l'éthanol.

### Spectre IR (CHCl₃) :

Absorptions à 1735 cm⁻¹ (-OAc) ; 1708 cm⁻¹ (11 et 20 céto) ; 1668 et 1618 cm⁻¹ (Δ4,3-one).

### Spectre RMN (CDCl₃, 250 MHz, ppm) :

0,63 (s) : 18-CH₃ ; 1,42 (s) : 19-CH₃ ; 2,03 (s) et 2,15 (s): CO-CH₃ ; 5,74 (s) : H₄.

### EXEMPLE 3 : 17α-hydroxy 21-acétoxy pregn-4-èn-3,11,20-trione. STADE A : 9α-bromo 11β,17α-dihydroxy 21-acétoxy pregn-4-èn-3,20-dione.

On mélange sous gaz inerte 4,71 g de 17α-hydroxy 21-acétoxy pregna 4,9(11)-dièn-3,20-dione et 65 cm³ de tétrahydrofuranne puis ajoute à 0°/-5°C 3,26 g de N-bromosuccinimide, puis, lentement, un mélange de 2,6 cm³ d'acide perchlorique à 70% et 5,2 cm³ d'eau. Après 1 heure 15 minutes sous agitation à 0°/-5°C, on verse la solution sur 50 volumes d'un mélange eau-glace, filtre, lave les cristaux à l'eau puis les sèche. On obtient 5,89 g de produit attendu.

### Spectre IR (CHCl₃) :

Absorptions à 1743-1722 cm⁻¹ (-CO-CH₂OAc) ; 1628 cm⁻¹ (Δ4, 3-céto) ; Absorption complexe région (OH/NH).

### STADE B : 17α-hydroxy 21-acétoxy pregn-4-èn-3,11,20-trione.

On mélange sous gaz inerte 0,7 g de produit obtenu au stade A 4,9 cm³ d'acétate d'éthyle et 1,6 cm³ d'éthylène glycol. On porte au reflux pendant 5 heures 15 minutes puis refroidit à 20°C et ajoute 1,1 cm³ d'acide chlorhydrique concentré et 12,2 cm³ d'eau. On maintient sous agitation pendant 16 heures puis distille l'acétate d'éthyle sous pression réduite. On sature la phase aqueuse au chlorure de sodium puis essore les cristaux, les lave à l'eau saturée de chlorure de sodium puis les sèche. On reprend le produit par un mélange chloroforme-isopropanol (97-3), filtre et chromatographie le filtrat sur silice en éluant au mélange chloroforme-isopropanol (97-3). On obtient 0,23 g de produit attendu.

### Spectre IR (CHCl₃) :

Absorptions à 3610 cm⁻¹ (OH) ; 1748, 1730, 1707 et 1667 cm⁻¹ (C=O).

### EXEMPLE 4 : 17α,21-dihydroxy pregn-4-èn-3,11,20-trione (cortisone).

### STADE A : 9α-bromo 11β,17α,21-trihydroxy pregn-4-èn-3,20-dione.

On mélange sous gaz inerte à 0°/-5°C, 4,2 g de 17a,21-dihydroxy pregna-4,9(11)-dièn-3,20-dione et 46 cm³ de tétra-hydrofuranne. On y ajoute 3,26 g de N-bromo succinimide puis, lentement, un mélange de 2,6 cm³ d'acide perchlorique à 70% et 5,2 cm³ d'eau. Après 1 heure 15 minutes à 0°/-5°C, on verse la solution sur 50 volumes d'un mélange eau-glace. On filtre, lave les cristaux à l'eau et les sèche. On obtient 4,86 g de produit attendu.

### Spectre IR (Nujol) :

Absorptions à 1710 cm⁻¹ (cétone non conjuguée), 1663 et 1618 cm⁻¹ (cétone conjuguée), Absorption région OH/NH.

### STADE B : 17α,21-dihydroxy pregn-4-èn-3,11,20-trione.

On mélange sous gaz inerte 0,7 g du produit obtenu au stade A, 4,9 cm³ d'acétate d'éthyle et 1,6 cm³ d'éthylène glycol. On porte au reflux pendant 5 heures 15 minutes puis refroidit à 20°C et ajoute 1,1 cm³ d'acide chlorhydrique concentré et 12,2 cm³ d'eau. On maintient sous agitation pendant 16 heures puis distille l'acétate d'éthyle sous pression réduite. On sature au chlorure de sodium puis extrait au chlorure de méthylène, lave la phase organique avec une solution aqueuse saturée de bicarbonate de sodium, puis avec une solution aqueuse saturée de chlorure de sodium, sèche et concentre à sec. On chromatographie le résidu sur silice en éluant au mélange chloroforme-isopropanol (92,5-7,5). On obtient 0,11 g de produit attendu que l'on peut purifier par dissolution dans un mélange chlorure de méthylène-éther isopropylique et distillation du chlorure de méthylène. Le produit attendu cristallise.

### Spectre IR (Nujol) :

Absorptions à 3480 cm⁻¹ (OH) ; 1700 cm⁻¹ (11 et 20 céto) ; 1650 cm⁻¹ (cétone conjuguée) ; 1613 cm⁻¹ (C=C).

## Revendications

1. Procédé de préparation des composés de formule (I) : 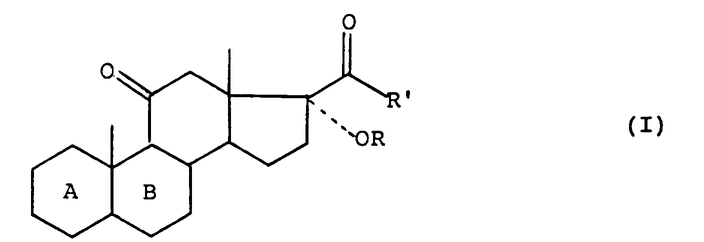 dans laquelle R représente un atome d'hydrogène ou un reste d'ester ou d'éther, R' représente un radical méthyle ou un radical -CH₂OR'', R'' représentant un reste d'ester, d'hydrogène ou d'éther identique ou différent de R, et les cycles A et B représentent un reste : 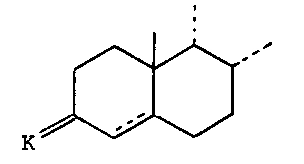 dans lequel K représente un atome d'oxygène ou un groupement protecteur du radical 3-oxo, de formule 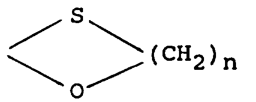 ou 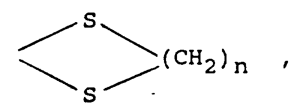 dans laquelle n est égal à 2 ou 3 et le trait pointillé représente une éventuelle seconde liaison, qui consiste à transformer un composé de formule (II) : 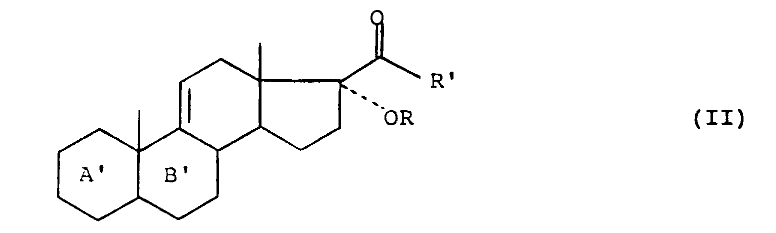 dans laquelle R et R' sont définis comme précédemment et les cycles A' et B' représentent soit les cycles A et B tels que définis précédemment, soit l'un des restes suivants : 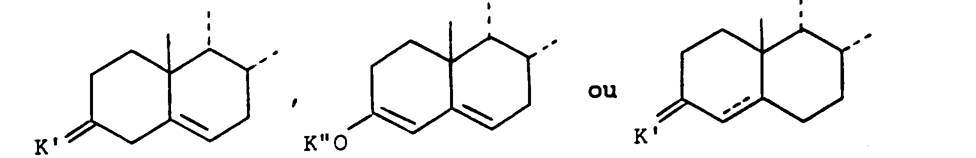 K' représentant un groupement protecteur de formule 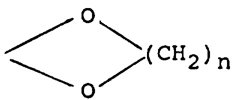 dans laquelle n est défini comme précédemment et K'' représente un radical alkyle renfermant de 1 à 8 atomes de carbone ou un radical aryle renfermant de 6 à 12 atomes de carbone, en une halohydrine de formule (III) : 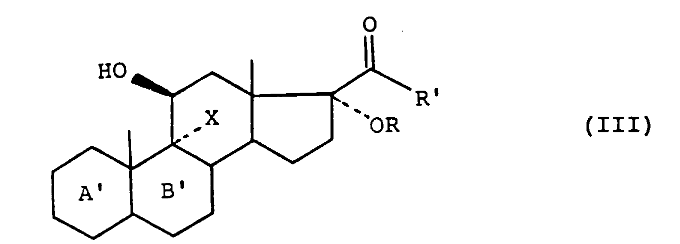 dans laquelle X représente un atome d'halogène et R, R', A' et B' sont définis comme précédemment et est caractérisé en ce que l'on soumet ladite halohydrine à une réaction de réarrangement en présence d'un alcool, pour obtenir après traitement par un acide le composé de formule (I) attendu.

2. Procédé selon la revendication 1, caractérisé en ce que la réaction de réarrangement est effectuée en présence d'un alcool supérieur ou d'un polyalcool, utilisé en excès.

3. Procédé selon la revendication 1 ou 2, caractérisé en ce que l'alcool supérieur ou le polyalcool est choisi dans le groupe constitué par le glycérol, l'éthylène glycol et le propylène glycol.

4. Procédé selon l'une quelconque des revendications 1 à 3, caractérisé en ce que l'on opère en présence d'éthylène glycol.

5. Procédé selon l'une quelconque des revendications 1 à 4, caractérisé en ce que l'on opère le réarrangement par chauffage à une température inférieure à 100°C.

6. Procédé selon l'une quelconque des revendications 1 à 5, caractérisé en ce que l'on opère en présence d'un cosolvant, inerte dans les conditions de la réaction, de point d'ébullition inférieur à 100°C et au reflux de celui-ci.

7. Procédé selon la revendication 6, caractérisé en ce que le cosolvant est l'acétate d'éthyle.

8. Procédé selon l'une quelconque des revendications 1 à 7, caractérisé en ce que l'on met en oeuvre une halohydrine de formule (III) dans laquelle X représente un atome de brome.

9. Procédé selon l'une quelconque des revendications 1 à 8, caractérisé en ce que l'on met en oeuvre une halohydrine de formule (III) dans laquelle les cycles A' et B' représentent un reste de formule : 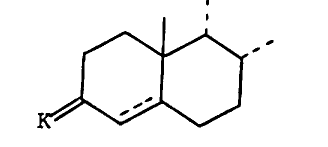 dans laquelle K et le trait pointillé en 4(5) sont définis comme à la revendication 1.

10. Procédé selon l'une quelconque des revendications 1 à 9, caractérisé en ce que l'on met en oeuvre une halohydrine de formule (III) dans laquelle R est choisi dans le groupe constitué par un atome d'hydrogène, un radical acyle renfermant de 1 à 8 atomes de carbone, un radical alkyle renfermant de 1 à 6 atomes de carbone, un radical tétrahydropyranyle et un reste d'éther silylé et R' représente un radical méthyle ou un radical -CH₂-OR'', R'' identique ou différent de R, ayant l'une quelconque des valeurs de R indiquées ci-dessus.

## Patentansprüche

1. Verfahren zur Herstellung von Verbindungen der Formel (I) 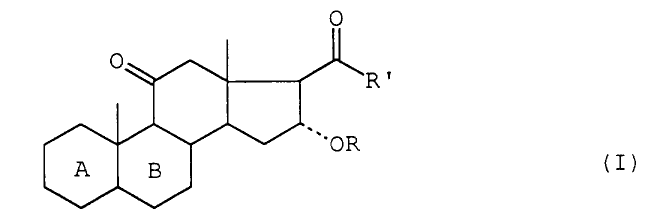 in der R ein Wasserstoffatom oder einen Ester- oder Etherrest darstellt, R' einen Methylrest oder einen Rest -CH₂OR" bedeut t, R" einen mit R gleichen oder von R verschiedenen Ester-wasserstoff, oder Etherrest darstellt, und die Ringe A und B einen Rest 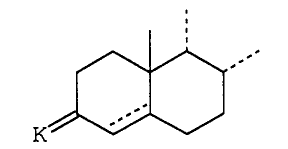 darstellen, worin K ein Sauerstoffatom oder eine Schutzgruppe des Restes 3-Oxo der Formel 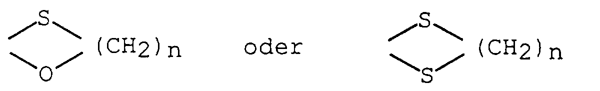 bedeutet, worin n gleich 2 oder 3 ist und die punktierte Linie eine eventuelle zweite Bindung bedeutet,
das darin besteht, daß man eine Verbindung der Formel (II) 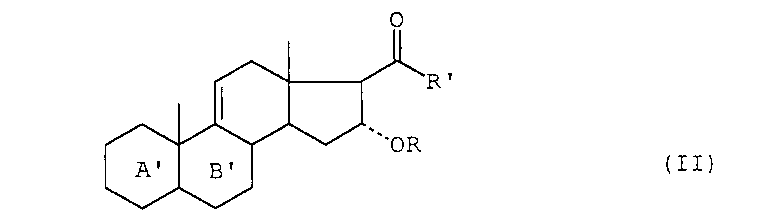 in der R und R' wie oben definiert sind und die Ringe A' und B' die wie oben definierten Ringe A und B sind, die einen der folgenden Reste bedeuten: 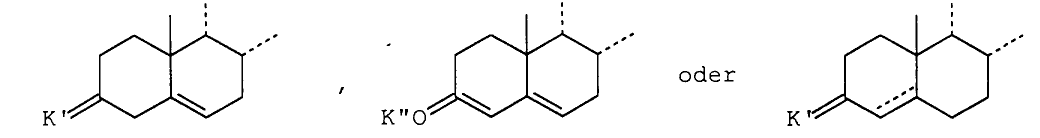 worin K' eine Schutzgruppe der Formel 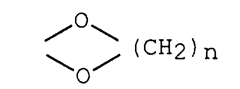 darstellt, in der n wie oben definiert ist und K" einen Alkylrest mit 1 bis 8 Kohlenstoffatomen oder einen Arylrest mit 6 bis 12 Kohlenstoffatomen bedeutet, in ein Halohydrin der Formel (III) 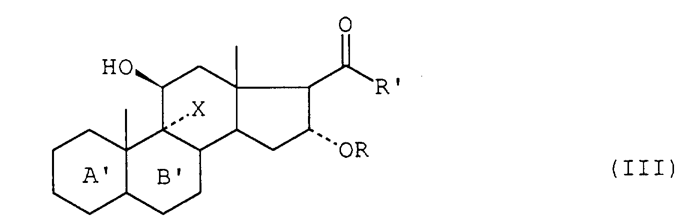 umwandelt, in der X ein Halogenatom darstellt und R, R', A' und B' wie oben definiert sind, und dadurch gekennzeichnet, daß man das genannte Halohydrin einer Umlagerungsreaktion in Anwesenheit eines Alkohols unterzieht, um nach Behandlung mit einer Säure die erwartete Verbindung der Formel (I) zu erhalten.

2. Verfahren nach Anspruch 1, dadurch gekennzeichnet, daß die Umlagerungsreaktion in Anwesenheit eines höheren Alkohols oder eines Polyalkohols, im Überschuß angewendet, durchgeführt wird.

3. Verfahren nach Anspruch 1 oder 2, dadurch gekennzeichnet, daß der höhere Alkohol oder der Polyalkohol aus der durch Glycerin, Ethylenglycol und Propylenglycol gebildeten Gruppe gewählt wird.

4. Verfahren nach irgendeinem der Ansprüche 1 bis 3, dadurch gekennzeichnet, daß man in Anwesenheit von Ethylenglycol arbeitet.

5. Verfahren nach irgendeinem der Ansprüche 1 bis 4, dadurch gekennzeichnet, daß man die Umlagerung durch Erhitzung auf eine Temperatur von unterhalb 100 °C durchführt.

6. Verfahren nach irgendeinem der Ansprüche 1 bis 5, dadurch gekennzeichnet, daß man in Anwesenheit eines Co-Lösungsmittels arbeitet, das unter den Reaktionsbedingungen, dem Siedepunkt von unterhalb 100 °C und unter dessen Rückfluß inert ist.

7. Verfahren nach Anspruch 6, dadurch gekennzeichnet, daß das Co-Lösungsmittel Ethylacetat ist.

8. Verfahren nach irgendeinem der Ansprüche 1 bis 7, dadurch gekennzeichnet, daß man ein Halohydrin der Formel (III) einsetzt, in der X ein Bromatom darstellt.

9. Verfahren nach irgendeinem der Ansprüche 1 bis 8, dadurch gekennzeichnet, daß man ein Halohydrin der Formel (III) einsetzt, in der die Ringe A' und B' einen Rest der Formel 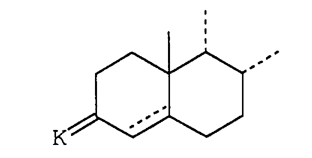 darstellen, in der K und die punktierte Linie in 4(5) wie in Anspruch 1 definiert sind.

10. Verfahren nach irgendeinem der Ansprüche 1 bis 9, dadurch gekennzeichnet, daß man ein Halohydrin der Formel (III) einsetzt, in der R aus der durch ein Wasserstoffatom, einen Acylrest mit 1 bis 8 Kohlenstoffatomen, einen Alkylrest mit 1 bis 6 Kohlenstoffatomen, einen Tetrahydropyranylrest und einen silylierten Etherrest gebildeten Gruppe gewählt wird und R' einen Methylrest oder einen Rest -CH₂-OR" bedeutet, worin R" gleich mit R oder verschieden von R ist, mit irgendeinem der oben angegebenen Werte von R.

## Claims

1. Preparation process for compounds of formula (I): 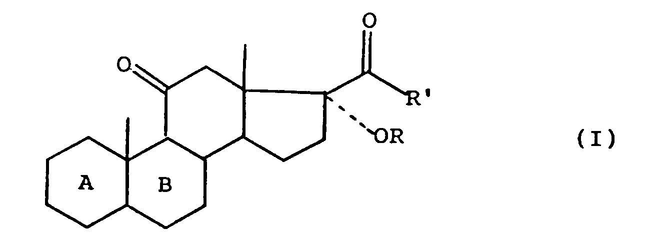 in which R represents a hydrogen atom or an ester or ether remainder, R' represents a methyl radical or a -CH₂OR" radical, R" representing an ester hydrogen or ether remainder identical to or different from R, and rings A and B represent a remainder: 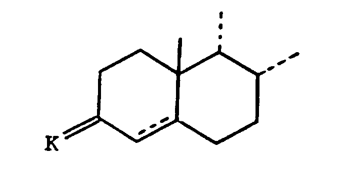 in which K represents an oxygen atom or a protector group of the 3-oxo radical of formula 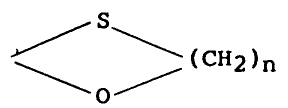 or 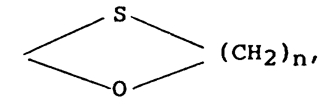 in which n is equal to 2 or 3 and the dotted line represents an optional second bond, which consists of converting a compound of formula (II): 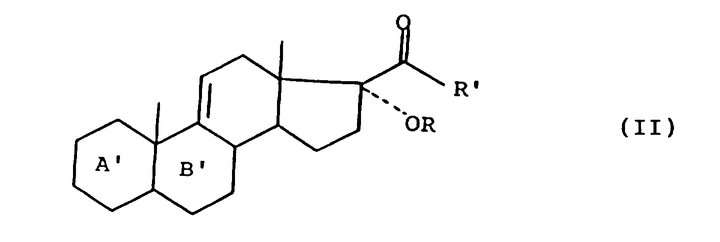 in which R and R' are defined as previously and rings A' and B' represent either rings A and B as defined previously, or one of the following remainders: 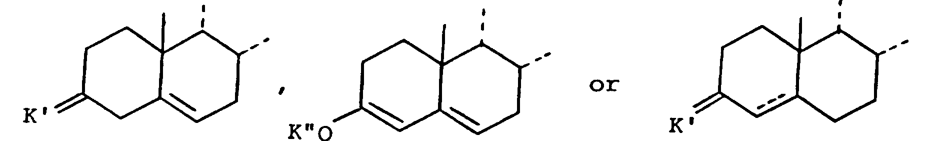 K' representing a protector group of formula 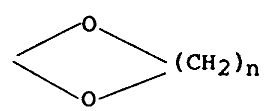 in which n is defined as previously and K" represents an alkyl radical containing 1 to 8 carbon atoms or an aryl radical containing 6 to 12 carbon atoms, into a halohydrin of formula (III): 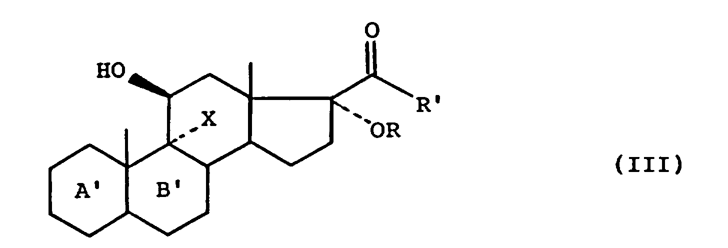 in which X represents a halogen atom and R, R', A' and B' are defined as previously, and is characterized in that the said halohydrin is subjected to a rearrangement reaction in the presence of an alcohol, in order to obtain after treatment by an acid the expected compound of formula (I).

2. Process according to claim 1, characterized in that the rearrangement reaction is carried out in the presence of a higher alcohol or a polyalcohol, used in excess.

3. Process according to claim 1 or 2, characterized in that the higher alcohol or the polyalcohol is chosen from the group constituted by glycerol, ethylene glycol and propylene glycol.

4. Process according to any one of claims 1 to 3, characterized in that the operation is carried out in the presence of ethylene glycol.

5. Process according to any one of claims 1 to 4, characterized in that the rearrangement is carried out by heating to a temperature lower than 100°C.

6. Process according to any one of claims 1 to 5, characterized in that the operation is carried out in the presence of a cosolvent, which is inert under the reaction conditions, and which has a boiling point lower than 100°C and under reflux of this cosolvent.

7. Process according to claim 6, characterized in that the cosolvent is ethyl acetate.

8. Process according to any one of claims 1 to 7, characterized in that a halohydrin of formula (III) is used in which X represents a bromine atom.

9. Process according to any one of claims 1 to 8, characterized in that a halohydrin of formula (III) is used in which the rings A' and B' represent a remainder of formula: 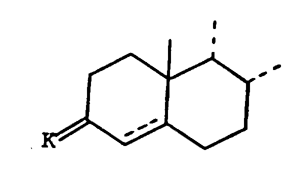 in which K and the dotted line in position 4(5) are defined as in claim 1.

10. Process according to any one of claims 1 to 9, characterized in that a halohydrin of formula (III) is used in which R is chosen from the group constituted by a hydrogen atom, an acyl radical containing 1 to 8 carbon atoms, an alkyl radical containing 1 to 6 carbon atoms, a tetrahydropyranyl radical and a silylated ether remainder and R¹ represents a methyl radical or a -CH₂-OR" radical, R" being identical to or different from R and having any one of the values of R indicated above.
